(19) Europäisches Patentamt
European Patent Office
Office européen des brevets

(11) EP 2 889 290 A1

# (12) EUROPEAN PATENT APPLICATION

(43) Date of publication:
**01.07.2015 Bulletin 2015/27**

(21) Application number: **13006056.9**

(22) Date of filing: **28.12.2013**

(51) Int Cl.:
***C07D 239/04*** *(2006.01)* ***C07D 295/15*** *(2006.01)*
***C23F 11/14*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Clariant International Ltd.**
**4132 Muttenz (CH)**

(72) Inventors:
- **Leinweber, Dirk**
  **65779 Kelkheim (DE)**
- **Benson, Hannah**
  **64646 Heppenheim (a.d. Bergstrasse) (DE)**
- **Kirkpatrick, Alistair**
  **Aberdeen, Scotland**
  **AB25 2JS (GB)**

(74) Representative: **Mikulecky, Klaus et al**
**Clariant Produkte (Deutschland) GmbH**
**Patent & License Management Chemicals**
**Industriepark Höchst, G 860**
**65926 Frankfurt am Main (DE)**

## (54) N-Alkyl-N'-poly(oxyalkyl)hexahydropyrimidine-betaines and the use thereof as corrosion inhibitors

(57) This invention relates to N-alkyl-N'-poly(oxyalkyl)hexapyrimidine- betaines of the formulae (Ia) - (Ic) and mixtures thereof

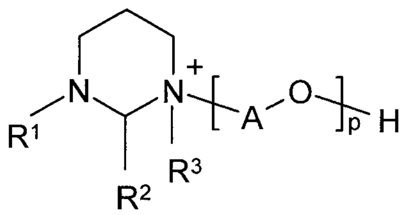

(Ia)

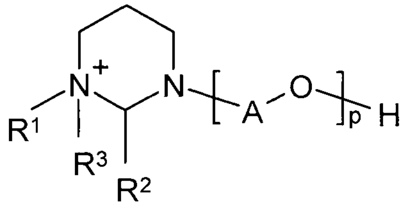

(Ib)



Printed by Jouve, 75001 PARIS (FR)

(Cont. next page)

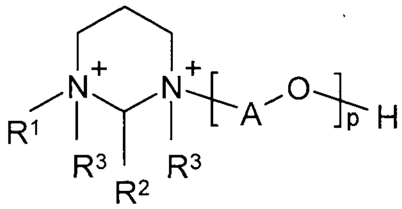

(Ic)

in which

$R^1$

is $C_8$-$C_{30}$-alkyl or $C_8$-$C_{30}$-alkenyl,

$R^2$

is hydrogen, $C_1$-$C_3$-alkyl, -COOH or a group selected from the formulae

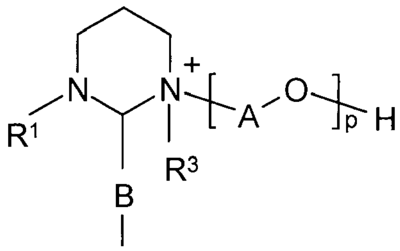

(Id)

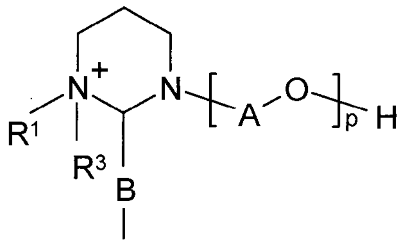

(Ie)

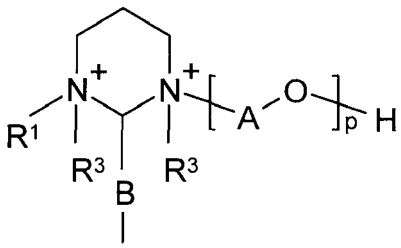

(If)



(Cont. next page)

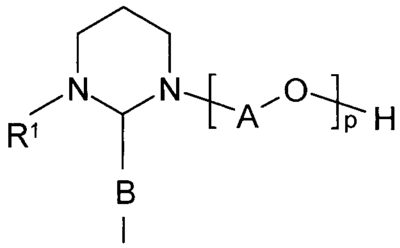

(Ig)

wherein

the bonding occurs via the valence containing the B residue,

B

is a single bond or a $C_1$ to $C_3$ alkylene group

$R^3$

is $-CH_2COO^-$ or $-CH_2CH_2COO^-$

A

is a 1,2-alkylene group having from 2 to 10 carbon atoms, and

p

is a number from 1 to 50.

## Description

Corrosion is a serious and challenging problem in the oil and gas industry and its prevention is acute in offshore operations. Water, acidic gases such as hydrogen sulfide and carbon dioxide, organic acids, and oxygen contribute to the corrosion of mild steel, and other types of alloys used in the oil and gas industry. Corrosion can cause oil and gas to leak from flowlines which can lead to explosions, accidents, and environmental disasters. Corrosion inhibitors are essential for preventing uncontrolled discharge of oil and /or gas into the environments surrounding the flowlines.

Corrosion inhibitors are either water-soluble or oil soluble chemical compounds. When added in small quantities to an aggressive medium, these chemicals inhibit corrosion by changing the surface conditions of the metal. Factors like $CO_2$, $H_2S$, S, polysulfides, organic acids, composition of liquids, flow conditions, inorganic anions, such as chlorides, oxygen, and temperature have an influence on the corrosion rate.

Zwitterionics such as betaines have long been known as Film-Forming Corrosion Inhibitors (FFCIs) (see: Production of Chemicals for the Oil and Gas Industry, Malcolm A. Kelland, 2009, CRC Press Taylor & Francis Group).

US 6171521 discloses zwitterionic water-soluble substituted imine corrosion inhibitors.

WO 2004/092447 describes a series of novel zwitterionic acrylated imidazolines for the use as corrosion inhibitors.

US2004/0169161 A1 discloses the use of doubly alkoxylated quaternary compounds as corrosion inhibitors with improved water solubility and improved film persistence. US-005530131A describes N-alkyl-N'-poly(oxyalkyl)-hexahydropyrimidines of the formula (X)

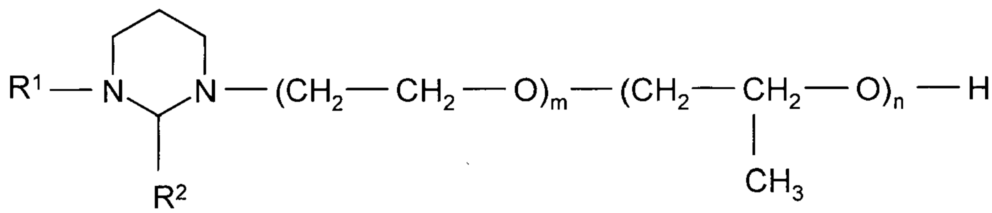

(X)

in which:

| | |
|---|---|
| $R^1$ | is $C_1$-$C_{30}$-alkyl or $C_2$-$C_{30}$-alkenyl, |
| $R^2$ | is hydrogen or $C_1$-$C_3$-alkyl, |
| A | is a 1,2-alkylene group having from 2 to 10, preferably from 2 to 5, carbon atoms and |
| m | is a number from 0 to 50 |
| n | is a number from 0 to 50, |
| m+n | is between 1 and 50, and |

further provides for the use of N-alkyl-N'-poly(oxyalkyl)hexahydropyrimidines of the formula (X) as corrosion inhibitors in water/oil emulsions as are present in petroleum.

The problem to be solved was to synthesize improved corrosion inhibitors particularly suitable for sweet gas environments. For the purpose of this invention, the term "sweet gas" is meant to encompass natural gas which is essentially free of $H_2S$, but contains $CO_2$. The expression "essentially free" means that any $H_2S$ contained in sweet gas is of a concentration so low that it will not cause corrosion with metal that is in contact with the sweet gas. In said sweet gas, any metal corrosion is due to other components, for example due to $CO_2$.

It has now been found that use of N-alkyl-N'-poly(oxyalkyl)hexahydropyrimidine-betaines gives excellent corrosion-protection for water/oil emulsions as they are present in crude oil. Particularly in sweet gas environments, these compounds show improved corrosion inhibition properties when compared with conventional sweet gas corrosion inhibitors such as Coco benzyldimethylammonium chloride. A sweet gas environment with respect to crude oil means that the crude oil contain sweet gas as defined above. In such environment, sweet gas corrosion occurs from reaction of $CO_2$ with iron.

The invention provides N-alkyl-N'-poly(oxyalkyl)hexahydropyrimidine-betaines of the formulae (Ia) - (Ic)

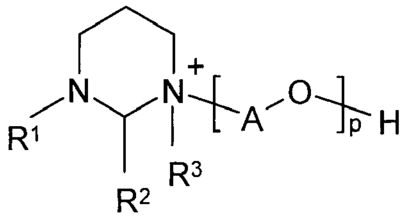

(Ia)

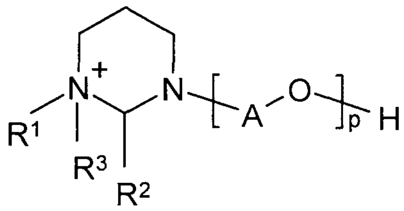

(Ib)

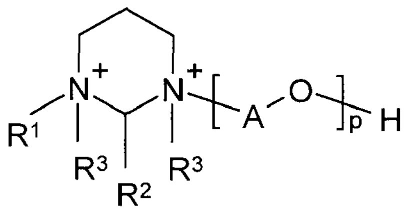

(Ic)

in which

$R^1$ is $C_8$-$C_{30}$-alkyl or $C_8$-$C_{30}$-alkenyl,

$R^2$ is hydrogen, $C_1$-$C_3$-alkyl, -COOH or a group selected from the formulae

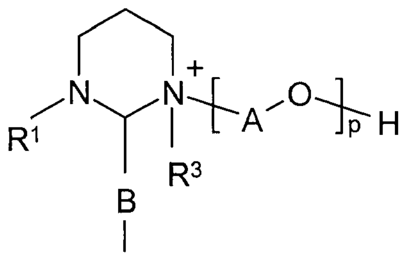

(Id)

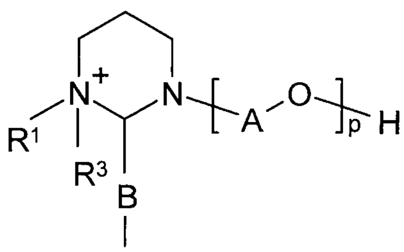

(Ie)

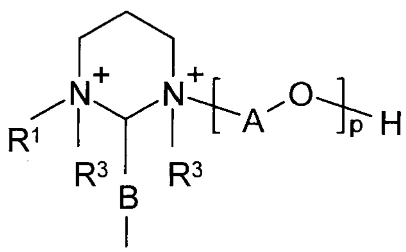

(If)

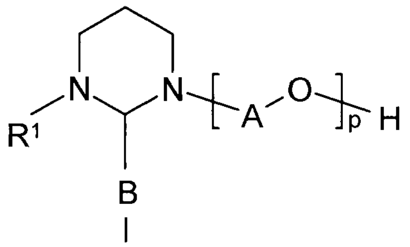

(Ig)

wherein
the bonding occurs via the valence containing the B residue,
B is a single bond or a $C_1$ to $C_3$ alkylene group,
$R^3$ is $-CH_2COO^-$ or $-CH_2CH_2COO^-$
A is a 1,2-alkylene group having from 2 to 10 carbon atoms and
p is a number from 1 to 50.

In another aspect of the invention, there is provided the use of one or more of compounds of formulae (Ia) - (Ic) as a corrosion inhibitor. Such use is preferably performed during the production and/or processing of crude oil and natural gas, particularly in the presence of sweet gas.

In another aspect of the invention there is provided a process for inhibiting corrosion of metal. The process comprises bringing the metal into contact with one or more of the compounds according to the formulae (Ia) - (Ic). The process is preferably applied to metal which is in contact with sweet gas during crude oil or natural gas production or processing.

Depending on the origin of the primary amine used in the synthesis of the compound (I), $R^1$ is preferably a radical of a naturally occurring fatty acid. Since the amines which are used in the synthesis of the compounds (Ia) to (Ic) and in which $R^1$ is an alkyl or alkenyl group are generally random mixtures of homologs and also of isomers, $R^1$ will usually be a mixture of different alkyl and/or alkenyl groups having various chain lengths. The number of carbon atoms given for $R^1$ shall therefore be understood as an average number.

Preference is given to compounds (I) in which $R^1$ is an alkyl or alkenyl group having from 8 to 24 carbon atoms, in particular having from 10 to 18 carbon atoms, especially those having from 12 to 18 carbon atoms. Particularly advantageous radicals $R^1$ are those which can be traced back to the $C_{10}$ fraction, the $C_{10}/C_{12}$ fraction, the $C_{12}/C_{14}$, or the $C_{16}/C_{18}$ fractions of a primary amine.

Examples of straight-chain or branched alkyl and alkenyl groups $R^1$ which may be mentioned are: n-octyl, 2-ethylhexyl, n- and iso-nonyl, n- and iso-decyl, n-undecyl, n-dodecyl, n-tetradecyl, n-hexadecyl, n-octadecyl, oleyl, linoleyl, linolenyl and behenyl.

In case that $R^2$ has the meaning of formulae (1d) to (1g), the inventive compounds have a structure like e.g. (for B = single bond)

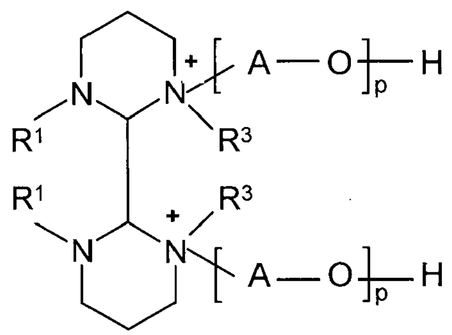

(Ih)

This is an exemplary structure. It is within the scope of this invention to have structures similar to formula (Ih) wherein $R^1$, $R^3$, A and p have different meanings in the two parts of the molecule linked by the B substituent.

A has preferably 2 to 5 carbon atoms. The 1,2-alkylene group A is preferably an ethylene group or a propylene, 1,2-butylene or 2,3-butylene group. Here, each group A can also be a random mixture of a plurality of the specified 1,2-alkylene groups, mixtures of ethylene and propylene units being preferred.

The degree of alkoxylation p is between 1 and 50, preferably from 3 to 35, in particular from 5 to 15. The values of p are usually averages.

$R^3$ is $-CH_2COO^-$ or-$CH_2CH_2COO^-$

The compounds of the formulae (Ia) -(Ic) of the invention are generally obtained by N-substitution of N-alkyl-N'-poly(oxyalkyl)-hexahydropyrimidines of the formula (II)

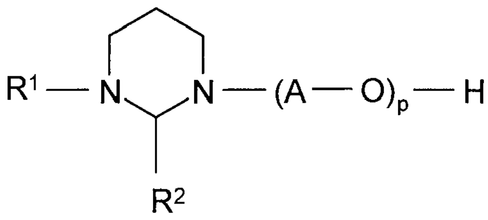
(II)

whose preparation is described in US-5530131A. The N-substitution of the compounds of the formula (II) is carried out by methods known per se.

Suitable methods are known, for example, from Jerry March, "Advanced organic chemistry" (John Wiley & Sons, 1985, 3rd edition).

Suitable reagents for N-substitution are for example sodium chloro acetate or acrylic acid. N-substitution by the reagents can occur via different mechanisms: 1) nucleophilic substitution in case the reagents contain a leaving group (e.g. chloride in sodium chloro acetate or 2) Michael addition in case the reagent contains a Michael-acceptor (e.g. the double bond in acrylic acid).

Preferable procedure for nucleophlic substitution reactions: N-alkyl-N'-poly(oxyalkyl)-hexahydropyrimidine and N-substitution reagent are reacted at temperatures in the range from 50 - 70 °C. Advantageously, the N-alkyl-N'-poly(oxyalkyl)-hexahydropyrimidine is initially charged, heated and the reagent is added. The reaction is exothermic and it should be avoided that the reaction mixture is heated to > 80 °C. The reaction mixture is stirred, preferably at 70 °C, in order to obtain complete conversion to the desired product of the formulae (Ia) - (Ic) or product mixture thereof.

Preferable procedure for Michael additions: N-alkyl-N'-poly(oxyalkyl)-hexahydropyrimidine and acrylic acid is reacted in at temperatures in the range from 100 - 130 °C. Advantageously, the N-alkyl-N'-poly(oxyalkyl)-hexahydropyrimidine is initially charged, and the reagent is added dropwise. The reaction mixture is stirred, preferably at 120 °C, in order to obtain complete conversion to the desired product of the formulae (Ia) - (Ic) or product mixture thereof.

If an equimolar amount or less than an equimolar amount of reagent for N-substitution is used, a mixture of starting material (N-alkyl-N'-poly(oxyalkyl)-hexahydropyrimidine) and substances of the formulae (Ia) - (Ic) is obtained. If more than an equimolar amount of N-substitution reagent is used, a mixture of substances of the formulae (Ia) - (Ic) is obtained. The expression equimolar means one mole reagent for N-substitution per mole of starting material.

If $R^2$ = H or $C_1$-$C_4$-alkyl and two equivalents of reagent for N-substitution are used, a nearly complete conversion to compound (Ic), with $R^2$ = H or $C_1$-$C_4$-alkyl is observed.

If $R^2$ = a group selected from the formulae (Id) - (Ig) and four equivalents of reagent for N-substitution are used, a nearly complete conversion to compound (Ic), with $R^2$ = (If) is observed.

To avoid byproducts, in particular oxidation products, the preparation of the substances of the formula (I) is preferably carried out under a stream of inert gas, preferably a stream of nitrogen. The products of the formulae (Ia) - (Ic) are generally obtained in good yield and with high degree of purity.

The substances of the formulae (Ia) - (Ic) and mixtures thereof are suitable as corrosion inhibitors, in particular in petroleum extraction and processing plants which come into contact with salt water. The amounts of these compounds used as corrosion inhibitors are from 1 to 200, preferably from 5 to 50 mg per liter of corrosive liquid. Since the compounds of the invention are usually prepared as highly viscous liquids, they are in practice normally used as a 20 - 50 % by weight strength solution, for example in water, glycols, glycol ethers, alcohols and other suitable solvents. These solutions can also include other corrosion-inhibiting active ingredients and also emulsifiers, antifoaming agents and further customary additives which improve the useful properties of the product being applied.

In general, however, the corrosion-inhibiting effect of such mixtures is provided by the corrosion-inhibitor components of the invention alone.

Preparative examples

In this specification, all percentages mean weight percent, unless otherwise noted.

Example 1

265.4 (0.5 mol) of N-coco alkyl-N'-poly(oxyalkyl)-(2-propyl-hexahydropyrimidine (A = EO, p = 5) and butyl glycol

(50 wt.-%) are initially charged in a flask with stirrer and reflux condenser under a stream of nitrogen. While stirring, 70 % aq. sodium chloro acetate (116.5 g sodium chloro acetate, 1.0 mol) is added within 10 minutes . After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 2

294.5 g (0.5 mol) of N-coco alkyl-N'-poly(oxyalkyl)-(2-methyl-hexahydropyrimidine (A = $(EO)_3(PO)_3$, p = 6) and butyl glycol (50 wt.-%) are initially charged in a flask with stirrer and reflux condenser under a stream of nitrogen. While stirring, 70 % aq. sodium chloro acetate (58.25 g sodium chloro acetate, 0.5 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 3

156.0 g (0.5 mol) of N-coco alkyl-N'-poly(oxyalkyl)-hexahydropyrimidine (A = EO, p = 1) and butyl glycol (50 wt.-%) are initially charged in a flask with stirrer and reflux condenser under a stream of nitrogen. While stirring, 70 % aq. sodium chloro acetate (29.12 g sodium chloro acetate, 0.25 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 4

381.6 g (0.5 mol) of N-tallow alkyl-N'-poly(oxyalkyl)-hexahydropyrimidine (A = EO, p = 10) and butyl glycol (50 wt.-%) are initially charged in a flask with stirrer and reflux condenser under a stream of nitrogen. While stirring, 70 % aq. sodium chloro acetate (116.5 g sodium chloro acetate, 1.0 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 5

381.6 g (0.5 mol) of N-tallow alkyl-N'-poly(oxyalkyl)-hexahydropyrimidine (A = EO, p = 10) and butyl glycol (50 wt.-%) are initially charged in a flask with stirrer and reflux condenser under a stream of nitrogen. While stirring, 70 % aq. sodium chloro acetate (58.25 g sodium chloro acetate, 0.5 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 6

200.3 g (0.5 mol) of N-coco alkyl-N'-poly(oxyalkyl)-(hexahydropyrimidine-2-carboxylic acid) (A = EO, p = 2) and butyl glycol (50 wt.-%) are initially charged in a flask with stirrer and reflux condenser under a stream of nitrogen. While stirring, 70 % aq. sodium chloro acetate (116.5 g sodium chloro acetate, 1.0 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 7

408.6 g (0.5 mol) of N-tall oil alkyl-N'-poly(oxyalkyl)-(2-propyl-hexahydropyrimidine) (A = EO, p = 10) and butyl glycol (50 wt.-%) are initially charged in a flask with stirrer and reflux condenser under a stream of nitrogen. While stirring, 70 % aq. sodium chloro acetate (116.5 g sodium chloro acetate, 1.0 mol) is added dropwise within 10 minutes. After the complete addition, the reaction mixture is stirred at 70 °C until the conversion is complete (monitored by titration of the

chloride value and by HPLC). After cooling to room temperature, the reaction mixture was filtered. The aqueous phase was separated from the organic phase in a separating funnel in order to obtain the product as clear brown liquid.

Example 8

691.9 g (0.5 mol) of N-lauryl-N'-poly(oxyalkyl)-(2-butyl-hexahydropyrimidine) (A = EO, p = 25) and butyl glycol (50 wt.-%) are initially charged in a flask with stirrer and reflux condenser under a stream of nitrogen. While stirring, 70 % aq. sodium chloro acetate (116.5 g sodium chloro acetate, 1.0 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 9

614.0 g (0.5 mol) of a N-alkyl-N'-poly(oxyalkyl)-(hexahydropyrimidine) of the formula

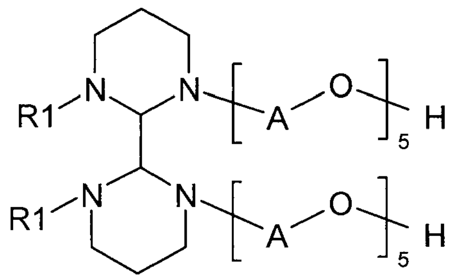

wherein

$R_1$ is behenyl, and
A is ethylene

with A = EO, p = 5, $R^1$ = behenyl, $R^2$ = I (g) with B = single bond, A = EO, p = 5,
$R^1$ = behenyl and butyl glycol (50 wt.-%) are initially charged in a flask with stirrer and reflux condenser under a stream of nitrogen. While stirring, 70 % aq. sodium chloro acetate (116.5 g sodium chloro acetate, 1.0 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 10

508.8 g (0.5 mol) of a N-alkyl-N'-poly(oxyalkyl)-(hexahydropyrimidine) of the formula

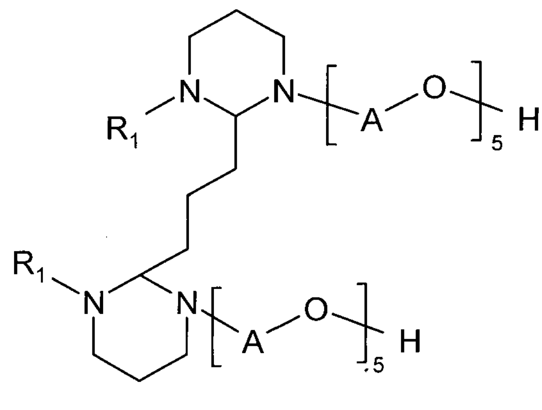

wherein

$R_1$ is derived from coco fatty acid ($C_{12}/C_{14}$), and
A is ethylene

with A = EO, p = 5, $R^1$ = C-chain derived from coco fatty acid, $R^2$ = I (g) with B = $C_3$-alkylene group, A = EO, p = 5, $R^1$ = C-chain derived from coco fatty acid and butyl glycol (50 wt.-%) are initially charged in a flask with stirrer and reflux condenser under a stream of nitrogen. While stirring, 70 % aq. sodium chloro acetate (116.5 g sodium chloro acetate, 1.0 mol) is added dropwise within 10 minutes. After the complete addition, the reaction mixture is stirred at 70 °C until the conversion is complete (monitored by titration of the chloride value and by HPLC). After cooling to room temperature, the reaction mixture was filtered. The aqueous phase was separated from the organic phase in a separating funnel in order to obtain the product as clear brown liquid.

Example 11

200.3 g (0.5 mol) of N-coco alkyl-N'-poly(oxyalkyl)-(hexahydropyrimidine-2-carboxylic acid) (A = EO, p = 2) are initially charged in a flask with stirrer and reflux condenser under a stream of nitrogen. While stirring, acrylic acid (36.03 g, 0.5 mol) is added dropwise within 10 minutes. After the complete addition, the reaction mixture is stirred at 120 °C until the conversion is complete (monitored by HPLC). After cooling to room temperature, the product was obtained as clear brown liquid.

Corrosion tests

Sweet LPR Bubble Testing

Linear Polarisation Resistance (LPR) bubble tests were conducted in 1 L Pyrex glass vessels containing 900 mL of synthetic brine (Brine composition listed in Table 1) that was continually purged with $CO_2$ gas whilst being heated to 80 °C. Once an oxygen content of less than 10 ppb was achieved, a probe was inserted into the fluid onto which were mounted CS 1018 working and auxiliary electrodes and a Hastelloy reference electrode.

The probes were connected to an ACM instruments potentiostat through a zero resistance ammeter and the LPR measured every fifteen minutes to determine the rate of corrosion of the CS 1018 working electrode. After two hours, 100 mL of kerosene (also de-aerated with $CO_2$) was carefully layered on top of the brine phase, ensuring no kerosene came into contact with the electrodes. After a further two hours, 10 ppm (based on total fluid volume of 1 L) of the chemical to be evaluated was added to the kerosene layer and the change in LPR, and therefore corrosion rate, recorded for twenty hours.

The percentage protection is calculated using the following formula;

$$\%\ \text{Inhibition} = \left(\frac{A - B}{A}\right) \times 100$$

wherein A is the corrosion rate with no inhibitor present and B is the corrosion rate with inhibitor present

Test Conditions:

| | |
|---|---|
| Gas Composition | 100 % $CO_2$ |
| Pressure | Ambient |
| Brine Composition | 900 mL Synthetic Brine (See table 1) |
| Dose Rate | 10 ppm |
| Electrodes | CS 1018 Carbon Steel, 600 grit finished, acetone rinsed working and auxiliary; Hastelloy C, acetone rinsed reference |
| Test Duration | 24 hours |
| Stir Rate | 150 rpm before addition of kerosene, 0 rpm after |
| Temperature | 80 °C |

Table 1: Brine composition

| Ion Species | North Sea brine [mg/L] |
|---|---|
| $Na^+$ | 29 370 |
| $K^+$ | 372 |
| $Mg^{2+}$ | 3 312 |
| $Sr^{2+}$ | 574 |
| $Ba^{2+}$ | 252 |
| $Cl^-$ | 52 360 |
| $HCO_3^-$ | 400 |
| $CH_3CO_2^-$ | 417 |

LPR screenings of multiple N-alkyl-N'-poly(oxyalkyl)hexahydropyrimidine- betaines were conducted. These results are listed in Table 2.

Table 2: LPR Test Results for some N-alkyl-N'-poly(oxyalkyl)hexahydropyrimidine-betaine derivatives

| Example | Corrosion Inhibitor | Corrosion rate 2 hours after dosing [mpy] | Corrosion rate at final 2 hours [mpy] | % protection 2 hours after dosing | % protection final 2 hours |
|---|---|---|---|---|---|
| 12 | Reference [1]; Coco benzyl quat (comparative example) | 206.85 | 278.16 | 11.91 | 0.17 |
| 13 | N-tallow alkyl-N'-poly(oxyalkyl)-hexahydropyrimidine (A = EO, p = 10) (comparative example) | 33.49 | 36.64 | 84.26 | 82.78 |
| 14 | Example 1 | 1.20 | 0.02 | 99.20 | 99.80 |
| 15 | Example 2 | 2.53 | 0.08 | 98.74 | 99.21 |
| 16 | Example 3 | 2.51 | 0.07 | 98.72 | 99.45 |
| 17 | Example 4 | 1.18 | 0.02 | 99.45 | 99.99 |
| 18 | Example 5 | 2.48 | 0.06 | 98.83 | 99.97 |
| 19 | Example 6 | 2.73 | 0.10 | 98.54 | 98.98 |
| 20 | Example 7 | 1.15 | 0.02 | 99.50 | 99.99 |
| 21 | Example 8 | 2.12 | 0.04 | 99.23 | 99.99 |
| 22 | Example 9 | 2.68 | 0.09 | 98.65 | 99.02 |
| 23 | Example 10 | 2.83 | 0.13 | 98.32 | 98.87 |
| 24 | Example 11 | 1.19 | 0.02 | 99.49 | 99.99 |
| [1] commercially available Coco benzyldimethylammonium chloride (CAS: 68424-85-1]. | | | | | |

Two known chemicals were used as comparative examples: Coco benzyldimethylammonium chloride is a known corrosion inhibitor.. A parent product (N-tallowalkyl-N'-poly(oxyalkyl)-hexahydropyrimidine (A = EO; p = 10) was used as another comparison. The N-alkyl-N'-poly(oxyalkyl)hexahydropyrimidine-betaine derivatives were tested against the benchmark products. The chemicals performed in a superior manner to the comparative chemicals.

The structure used in comparative examples 13 and 26 is as follows:

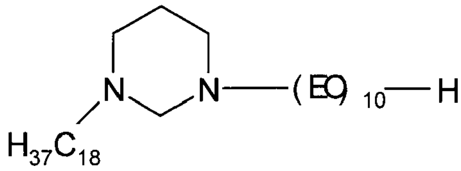

EO means the ethoxy unit.

Exemplary Formulation

Each formulated inhibitor consists of 20 % (by weight) of the active inhibitor molecule, 2 % Alkoxylated octyl ether carboxylic acid (Emulsogen CNO 080) and 2 % Thioalcohol (2-mercaptoethanol) with the remainder being a 50/50 mixture of Butylglycolether and water.

Table 3: LPR-test results of the reference compound/ the parent compound/ and an exemplary formulation

| Example | Corrosion Inhibitor | Corrosion rate 2 hours after dosing [mpy] | Corrosion rate at final 2 hours [mpy] | % protection 2 hours after dosing | % protection final 2 hours |
|---|---|---|---|---|---|
| 25 | Coco benzyldimethylammonium chloride formulated (comparative example) | 8.83 | 1.38 | 95.52 | 99.30 |
| 26 | N-tallowalkyl-N'-poly(oxyalkyl)-hexahydropyrimidine (A = EO; p = 10) formulated (comparative example) | 15.21 | 2.72 | 92.85 | 98.72 |
| 27 | Example 4 formulated | 3.54 | 0.16 | 98.18 | 99.92 |

## Claims

1. N-alkyl-N'-poly(oxyalkyl)hexahydropyrimidine-betaines of the formulae (Ia) - (Ic)

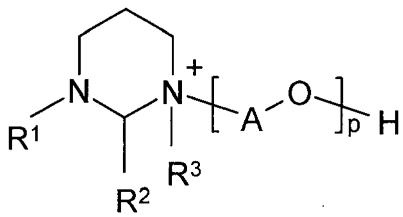

(Ia)

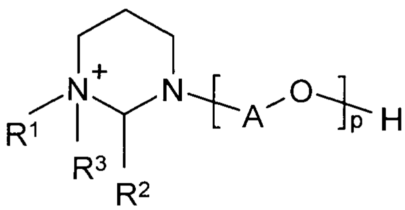

(Ib)

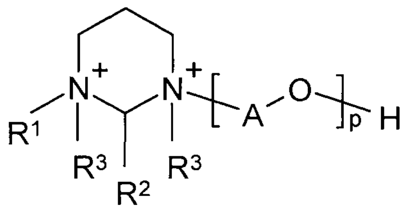

(Ic)

in which

$R^1$ is $C_8$-$C_{30}$-alkyl or $C_8$-$C_{30}$-alkenyl,
$R^2$ is hydrogen, $C_1$-$C_3$-alkyl, -COOH or a group selected from the formulae

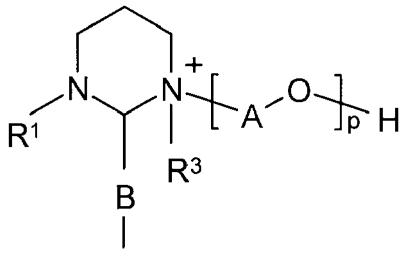

(Id)

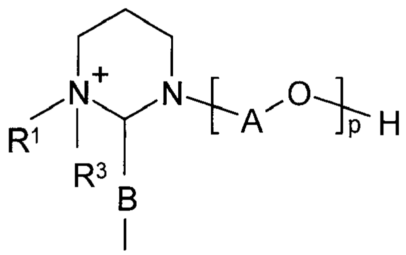

(Ie)

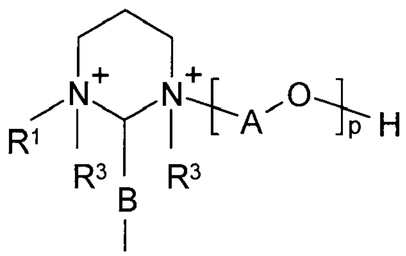

(If)

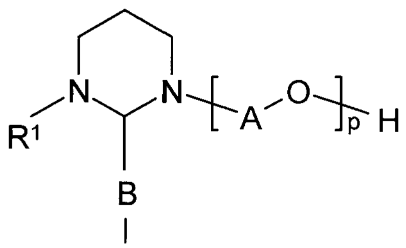

(Ig)

wherein
the bonding occurs via the valence containing the B residue,

B is a single bond or a $C_1$ to $C_3$ alkylene group
$R^3$ is $-CH_2COO^-$ or $-CH_2CH_2COO^-$

A is a 1,2-alkylene group having from 2 to 10 carbon atoms and
p is a number from 1 to 50.

2. Compounds according to claim 1, wherein $R^1$ is $C_{10}$-$C_{24}$-alkyl, or $C_{10}$-$C_{24}$-alkenyl.

3. Compounds according to claim 2, wherein $R^1$ is $C_{10}$-$C_{18}$-alkyl, or $C_{10}$-$C_{18}$-alkenyl.

4. Compounds according to one or more of claims 1 to 3, wherein $R^3$ is-$CH_2COO^-$.

5. Compounds according to one or more of claims 1 to 4, wherein $R^2$ is hydrogen.

6. Compounds as claimed in one or more of claims 1 to 5, wherein A is is a 1,2-alkylene group having from 2 to 5 carbon atoms.

7. Compounds as claimed in claim 6, wherein A is an ethylene group.

8. Compounds as claimed in one or more of claims 1 to 7, wherein p is between 3 to 35 on average.

9. Compounds as claimed in claim 8, wherein p is between 5 to 15 on average.

10. Compounds according to any one of claims 1 to 9 wherein formulae (Ia) - (Ic) are formula (Ih)

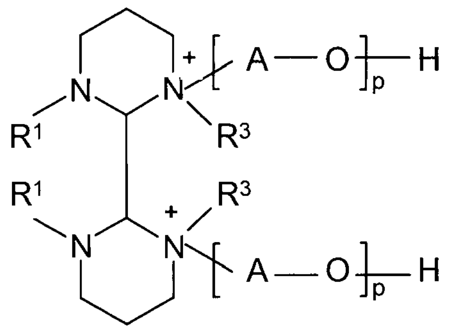

(Ih)

11. The use of compounds of one or more of claims 1 to 10 as corrosion inhibitors or in corrosion inhibitor-formulations.

12. A corrosion inhibitor formulation comprising at least one compound according to one or more of claims 1 to 10.

13. A process for inhibiting corrosion of metal, the process comprising bringing the metal into contact with one or more of the compounds according to one or more of claims 1 to 10.

Europäisches Patentamt
European Patent Office
Office européen des brevets

# EUROPEAN SEARCH REPORT

Application Number
EP 13 00 6056

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 0 625 512 A1 (HOECHST AG [DE]; HOFFMANN HERRMANN [DE]) 23 November 1994 (1994-11-23) * the whole document * ----- | 1-13 | INV. C07D239/04 C07D295/15 C23F11/14 |
| A | JP 2006 169595 A (CHUBU KIRESUTO KK; KIRESUTO KK) 29 June 2006 (2006-06-29) * the whole document * ----- | 1-13 | |
| A | WO 2009/064719 A1 (NALCO COMPANY [US]; NGUYEN DUY T [US]) 22 May 2009 (2009-05-22) * the whole document * ----- | 1-13 | |
| | | | TECHNICAL FIELDS SEARCHED (IPC) |
| | | | C07D C23F |

The present search report has been drawn up for all claims

1

| Place of search | Date of completion of the search | Examiner |
|---|---|---|
| Munich | 18 March 2014 | Ladenburger, Claude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 00 6056

This annex lists the patent family members relating to the patent documents cited in the above-mentioned European search report. The members are as contained in the European Patent Office EDP file on
The European Patent Office is in no way liable for these particulars which are merely given for the purpose of information.

18-03-2014

| Patent document cited in search report | Publication date | Patent family member(s) | Publication date |
|---|---|---|---|
| EP 0625512 A1 | 23-11-1994 | BR 9401971 A | 27-12-1994 |
| | | DE 4316374 A1 | 17-11-1994 |
| | | EP 0625512 A1 | 23-11-1994 |
| | | NO 941798 A | 16-11-1994 |
| | | RU 2126796 C1 | 27-02-1999 |
| | | US 5530131 A | 25-06-1996 |
| JP 2006169595 A | 29-06-2006 | JP 4824925 B2 | 30-11-2011 |
| | | JP 2006169595 A | 29-06-2006 |
| WO 2009064719 A1 | 22-05-2009 | AU 2008321109 A1 | 22-05-2009 |
| | | CA 2703883 A1 | 22-05-2009 |
| | | EP 2217673 A1 | 18-08-2010 |
| | | US 2009131283 A1 | 21-05-2009 |
| | | WO 2009064719 A1 | 22-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6171521 B
- WO 2004092447 A
- US 20040169161 A1
- US 005530131 A
- US 5530131 A


### Non-patent literature cited in the description


- **MALCOLM A. KELLAND.** Production of Chemicals for the Oil and Gas Industry. CRC Press Taylor & Francis Group, 2009
- **JERRY MARCH.** Advanced organic chemistry. John Wiley & Sons, 1985